# EUROPEAN PATENT APPLICATION

(11) **EP 2 601 983 A1**
(43) Date of publication of application: **12.06.2013**
(21) Application number: 12195952.2
(22) Date of filing: 06.12.2012
(51) Int. Cl.: A61M 1/00

(54) **Improvements in or relating to medical suction disposal system and components thereof**

(30) Priority: 08.12.2011 GB 201121257
(71) Applicant: VACSAX Limited, Plymouth, PL7 5BG (GB)
(72) Inventor: Bennett, John, Plymouth, Devon PL7 5BG (GB)
(74) Representative: Beatson, Matthew

(57) **Abstract**

A component (11) for a medical suction disposal system including a material having anti-microbial activity is provided. The component may be any part of the medical suction disposal system, such as for example a liner, a canister, a tube or a closure.

## Description

The present invention relates to a medical suction disposal system or a component thereof that at least includes or is coated with a material having anti-microbial activity.

Medical suction disposal systems are used to remove and contain medical waste so that it can be disposed of safely.

One suitable example of a medical suction disposal system is described in GB 2333459, where a disposable liner, positioned within a vacuum canister, is connected to a closure with suction line and vacuum connections.

Some medical suction disposal system components such as the above-identified liners contain recesses, folds, crevices or openings which could be suitable environments for microbial growth. This is especially true of the above-identified liner when it is produced in a collapsed configuration. This collapsed configuration may be due to the liner comprising a concertina fold as seen in GB 2333459 or a telescopic fold as seen in GB 2427555. The present invention seeks to address this issue, and to mitigate, or at least reduce, the growth of microbes in components of a medical suction disposal system especially, but not exclusively, one which contains such recesses, folds, crevices or openings.

Because of their use, medical suction disposal systems will inevitably come into contact with biological material which could allow for growth of microbes. This applies to any of the components of a medical disposal suction system, not only the liners. As such they all require to be sterilised separately after use, which can be burdensome.

The necessity for sterilisation is especially pertinent given the environment in which said disposal systems are generally used, such as hospital operating theatres, accident and emergency departments and dental, paediatric and recovery units.

The present invention seeks to overcome this problem, and to mitigate, or at least reduce, the need for repeated sterilisations of the components of a medical suction disposal system and/or to reduce the risk of such components harbouring microbes.

According to one aspect of the present invention there is provided a component for a medical suction disposal system including a material having anti-microbial activity.

The component may be any part of the medical suction disposal system, such as for example a liner, a canister, a tube or a closure.

The liner may be a disposable or reusable liner.

The liner may be in a collapsible configuration to provide advantages in storage and transport.

The tube may comprise or include the tubing from the site to be aspirated through to the vacuum machine.

The material having anti-microbial activity may be present as at least part of a layer on at least part of the component. The layer may be on the surface. The nature of the layer containing the anti-microbial activity may be determined by many factors which include the method of its application to the component, the function of the component, the amount of material having anti-microbial activity required and its concentration within the layer.

The material having anti-microbial activity may be at least part of the material that constitutes the component itself. The material which constitutes the component comprises a polymeric material and therefore the material having anti-microbial activity could be an additive and/or part of said polymeric material. The polymeric material may be polyolefin-based. For example, the polymeric material constituting the component may comprise or include one or more of the following polymers: polycarbonate, polypropylene or polyethylene.

Another aspect of the invention is a medical suction disposal system comprising one or more of the components as described hereinabove.

A further aspect of the invention is a medical suction disposal system in which at least part thereof is coated with material having anti-microbial activity. The nature of the coating depends on many factors, for example how it is applied to the suction device.

Likewise, the nature of the material having anti-microbial activity depends on many factors, such as whether it is applied to a surface of the suction device or whether it constitutes part of the suction device and of course whether it is targeted to a specific microbe, such as a specific type of bacterium, or whether it is required to have a more general microbial activity.

The material having anti-microbial activity may be anti-bacterial. The material may be biocidal. It may contain an active ingredient that is silver-based. The material may contain as active ingredient silver ions which are released over an extended period of time. The release over time may be achieved by using sequestering agents, such as chelators, to retain the silver ions over a period of time.

Another aspect of the invention is a method of producing a component for a medical suction disposal system which comprises the following steps:
- preparing a master-batch which comprises or includes material having anti-microbial properties, and
- using the master-batch to produce one or more of said components.

The method of production of said components from a master-batch may include injection moulding, blow moulding, compression moulding, vacuum forming, extrusion or rotational moulding.

Another aspect of the invention is a method of applying a material with anti-microbial activity to a medical suction disposal system or a component thereof which comprises the following steps:
- preparing a solution in which the material having anti-microbial activity is solubilized, and
- applying the solution to the medical suction disposal system or a component thereof.

Application of the solution may be effected by spraying the solution on to the medical suction disposal system or the component thereof or other suitable process such as dipping.

Another aspect of the invention is the use of a medical suction device or a component thereof as described above in removing and containing medical waste. This is particularly advantageous in situations where sanitation requirements are paramount, such as in in hospital operating theatres, accident and emergency departments and dental, paediatric and recovery units.

Further particular and preferred aspects and embodiments of the present invention are set out in the accompanying independent and dependent claims. Features of the dependent claims may be combined with features of the independent claims as appropriate, and in combinations other than those explicitly set out in the claims. Various embodiments of the invention will now be more particularly described, by way of example, with reference to the accompanying drawings, in which:
Figure 1 is a perspective view of a canister suitable for use in a medical suction disposal system in accordance with the invention; and
Figure 2 is a perspective view of a liner and a closure for use in a medical suction disposal system.

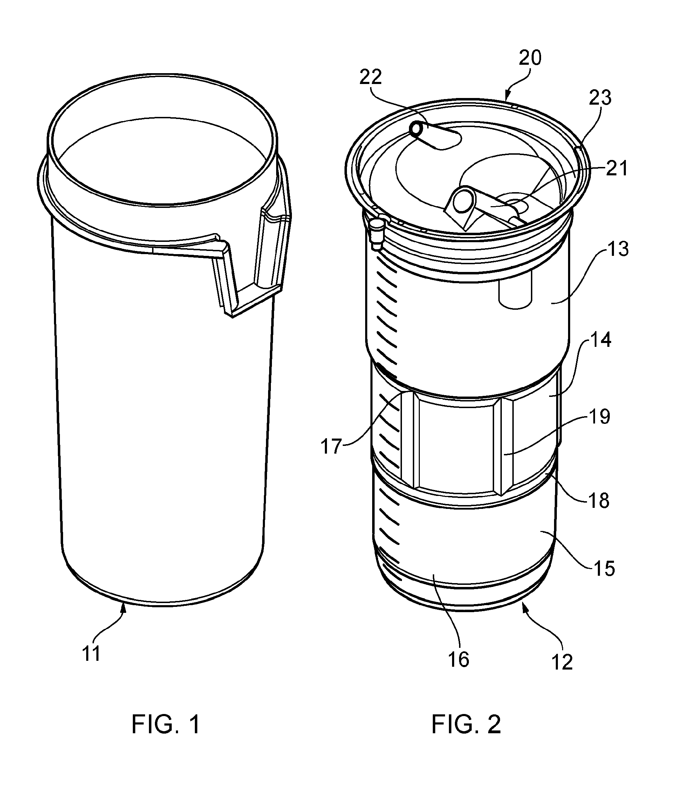

Referring now to the drawings, there is shown in Figure 1 a component for a medical suction disposal system in the form of a canister 11. The canister 11 is made of polymeric material that is transparent. Typically said polymeric material is made from a master-batch. In this embodiment of the invention the master-batch includes material having anti-microbial activities. The polymeric material needs to be rigid as the canister forms the backbone of the medical suction disposal system into which the liner expands under vacuum and thus must be able to hold the liner under vacuum. When a liner is present within the canister, the space between the two would be minimal and be suitable environment for microbial growth.

If the canister were made of, or contained polypropylene, the master-batch may, for example, be formed from antimicrobial master-batch material sold under the Registered Trade Mark Biomaster 542®. If, for example, the canister were formed from or contained polycarbonate, the master-batch may comprise or include antimicrobial master-batch material one sold under the Registered Trade Mark Biomaster 909®.

Figure 2 illustrates a telescopic liner 12. Liner 12 comprises a generally cylindrical flexible body 15 having a circular cross-section around its longitudinal axis. In this embodiment, the liner 12 is formed from a low density polyethylene, for example by blow moulding.

The liner 12 comprises three main sections: an upper fixed portion 13; a central rolling section 14 which is contiguous with the fixed portion 13; and a cup-like telescopic portion 15 which is contiguous with the rolling portion 14 and includes an inwardly domed base 16 defining a closed end of the liner. In this illustration, the liner is shown in its maximally extended position with the fixed portion 13, rolling portion 14 and telescopic portion 15 maximally spread along the longitudinal axis of the liner 12.

The fixed portion 13, rolling portion 14 and telescopic portion 15 are concentric, with the fixed portion 13 having the largest diameter and the telescopic portion 15 having the smallest diameter. The intersection between the fixed portion 13 and the rolling portion 14 is defined by a tapering strip in the form of a radially inclined step 17. Similarly, the rolling portion 14 is connected to the telescopic portion 15 by a radially inclined step 18. In some embodiments the steps 17, 18 are thinner than one or both of the walls of the portions flanking them, which assists in the folding operations described below.

In this embodiment, the rolling section 14 is provided with a plurality of circumferentially spaced, longitudinally extending grooves 19. The grooves 19 allow the rolling portion 14 to circumferentially expand and contract as it is folded/unfolded in use.

Attached to the fixed portion 13 is a closure 20 provided with a suction line connection 21 and a vacuum connection 22. The closure 20 includes a flange 23 around its upper edge to seat on the rim of a vacuum canister 11 in use, and is provided with the usual port (not shown) from the vacuum connection to the edge of the closure just below the flange 23 to evacuate the jar.

The liner 12 and closure 20 are made from polymeric material produced from a thermosetting resin master-batch which includes material having anti-microbial activity. If the closure or liner contained polypropylene and/or polypropylene, the master-batch may, for example, comprise or include antimicrobial material sold under the Registered Trade Mark Biomaster 542®. If the closure or liner were made of, or contained polycarbonate, the master-batch may, for example, comprise or include antimicrobial material sold under the Registered Trade Mark Biomaster 909®.

Although illustrative embodiments of the invention have been disclosed in detail herein, with reference to the accompanying drawings, it is understood that the invention is not limited to the precise embodiment and that various changes and modifications can be effected therein by one skilled in the art without departing from the scope of the invention as defined by the appended claims and their equivalents.

## Claims

1. A component for a medical suction disposal system including material having anti-microbial activity, in which the component is a liner, a canister or a closure.

2. A component as claimed in Claim 1, in which the material having anti-microbial activity is present as at least part of a layer on at least part of the component.

3. A component as claimed in Claim 1, in which the material having anti-microbial activity is at least part of the material that constitutes the component itself.

4. A component as claimed in Claim 3, in which the material that constitutes the component itself comprises a polymeric material, in which the polymeric material comprises one or more of the following polymers: polycarbonate, polypropylene or polyethylene.

5. A medical suction disposal system comprising one or more of the components as claimed in any preceding claim.

6. A medical suction disposal system in which at least part thereof is coated with material having an anti-microbial activity.

7. A component or medical suction device as claimed in any of the preceding claims, in which the material having anti-microbial activity includes silver.

8. A component or medical suction device as claimed in claim 7, in which the material having anti-microbial activity is one from which silver ions are released gradually over time.

9. A medical suction device or a component thereof substantially as hereinbefore described with reference to, and as shown in, the accompanying drawings.

10. A method of producing a component for a medical suction disposal system which comprises the following steps:
- preparing a master-batch which includes material having anti-microbial properties; and
- using the master-batch to produce one or more of the said components, wherein said one or more components is a liner, a canister or a closure.

11. A method as claimed in claim 10, in which the preparation of the said components from a master-batch includes at least one of the following steps: injection moulding, blow moulding, compression moulding, vacuum forming, extrusion or rotational moulding.

12. A method of applying a material with anti-microbial activity to a component for a medical suction disposal system which comprises the following steps:
- preparing a solution in which the material having anti-microbial activity is solubilized; and
- applying the solution to the component, wherein the components is a liner, a canister or a closure.

13. A method of producing a medical suction disposal system or a component thereof substantially as hereinbefore described with reference to, and as shown in, the accompanying drawings.

14. Use of a component or of a medical suction device according to any of claims 1 to 9 in removing and containing medical waste.

15. Use as claimed in claim 14, in which said use is carried out in hospital operating theatres, accident and emergency departments and dental, paediatric and recovery units.
